# EUROPÄISCHE PATENTANMELDUNG

(11) **EP 2 979 723 A1**
(43) Veröffentlichungstag der Anmeldung: **03.02.2016**
(21) Anmeldenummer: 15172365.7
(22) Anmeldetag: 16.06.2015
(51) Int. Cl.: A61M 25/06, A61M 25/00

(54) **EINFÜHRELEMENT**

(30) Priorität: 30.07.2014 US 201462030624 P
(71) Anmelder: Biotronik AG, 8180 Bülach (CH)
(72) Erfinder: Fargahi, Amir, 8180 Bülach (CH)
(74) Vertreter: Randoll, Sören

(57) **Zusammenfassung**

Die Erfindung betrifft ein Einführelement (100), welches zum Zusammenwirken mit einer Einführvorrichtung (10) zum Einführen eines medizinischen Implantats (20) in einen menschlichen oder tierischen Körper ausgebildet ist, und welches eine Hülle (102) mit einer Längsachse (114) aufweist. Ein Gerüst (110) mit wenigstens bereichsweise quer zur Längsachse (114) angeordneten und entlang der Längsachse (114) aufeinander folgenden Elementen (120) ist in die Hülle (102) integriert, wobei die Elemente (120) entlang der Längsachse (114) mit einem Längselement (130) verbunden sind, und welches Gerüst (110) eine höhere Härte aufweist als die Hülle (102).

Die Erfindung betrifft weiterhin eine Einführvorrichtung (10) mit einem solchen Einführelement (100).

## Beschreibung

Die Erfindung betrifft ein Einführelement nach dem Oberbegriff des Anspruchs 1.

In der Medizin kommen häufig Implantate zum Einsatz, die zur Erfüllung von Ersatzfunktionen permanent oder zumindest für einen längeren Zeitraum in einen menschlichen und/oder tierischen Körper eingebracht werden. Zu nennen wären hier beispielsweise Herzschrittmacher, Hirnschrittmacher für Parkinsonpatienten, Herzimplantate, Cochleaimplantate, Retina-Implantate, zahnmedizinische Implantate, Implantate zum Gelenkersatz, Gefäßprothesen oder Stents.

Implantate werden vor einem Einführen in den Körper mit Kathetern verbunden, mit dessen Hilfe sie am Einsatzort genau platziert und definiert freigegeben werden können. Zum Einschleusen in den menschlichen und/oder tierischen Körper wird hierzu ein schlauchartiges Einführelement eingesetzt, durch welches das Implantat mittels der Einführvorrichtung geschoben wird. Um die Belastung der Gefäße beim Einschleusen des Implantats zu vermindern, ist aus der US 2010/0094392 A1 ein Einführelement bekannt welches erst beim Passieren des Implantats auf den notwendigen größeren Durchmesser aufgeweitet wird. Das Einführelement besteht aus zwei bis drei koaxial angeordneten Lagen, wobei die äußere mit longitudinalen Schlitzen versehen ist, was die Vergrößerung des Durchmessers ermöglicht.

Die EP 2 676 641 A2 offenbart ein reversibel aufweitbares Einführelement mit einer Hülle auf, welche am Umfang Bereiche mit unterschiedlicher Elastizität aufweist. Ein Katheter kann durch das aufweitbare Einführelement in den Körper eingeführt werden, wobei das Einführelement sich beim Passieren des am Katheter befestigten Implantats aufweitet und nach Passieren wieder auf seinen ursprünglichen kleineren Durchmesser zusammenzieht. Der Erfindung liegt die Aufgabe zugrunde, ein Einführelement zu schaffen, das verbesserte mechanische Eigenschaften aufweist und zumindest reversibel biegsam ist.

Die Aufgabe wird erfindungsgemäß durch die Merkmale der unabhängigen Ansprüche gelöst. Günstige Ausgestaltungen und Vorteile der Erfindung ergeben sich aus den weiteren Ansprüchen, der Beschreibung und der Zeichnung.

Nach einem Aspekt der Erfindung wird ein Einführelement vorgeschlagen, welches zum Zusammenwirken mit einer Einführvorrichtung zum Einführen eines medizinischen Implantats in einen menschlichen und/oder tierischen Körper ausgebildet ist, und welches eine Hülle mit einer Längsachse aufweist. Ein Gerüst mit wenigstens bereichsweise quer zur Längsachse angeordneten und entlang der Längsachse aufeinander folgenden Elementen ist in die Hülle integriert, wobei die Elemente entlang der Längsachse mit einem Längselement verbunden sind, und welches Gerüst eine höhere Härte aufweist als die Hülle.

Vorteilhaft ist ein solches Einführelement als Schleuse für einen Katheter mit einem Implantat einsetzbar, über die der Katheter bzw. das Implantat in den Körper eingeführt werden kann. Das Einführelement weist durch das stabile Gerüst eine hohe Stabilität entlang seiner Längsachse auf, so dass einerseits eine vorteilhaft Biegsamkeit vorhanden ist und andererseits eine günstiges Verhalten bei Wirken einer Kompressionskraft, wenn der Katheter eingeschoben wird. Die gewünschten Eigenschaften können durch die Auswahl von günstigen Materialienkombination und deren Dimensionen optimiert werden.

Das Längselement unterstützt das Einführelement in Richtung der Längsachse, so dass das Einführelement bei Schubbewegungen oder Zugbewegungen des Katheters stabilisiert ist. Das Längselement kann mit den Elementen beispielsweise verschweißt, insbesondere mittels Laserschweißen verbunden sein. Die Elemente können Ringelemente sein, die insbesondere aufweitbar sein können. Dann ist das Einführelement zusätzlich auch radial aufweitbar. Die Elemente können jedoch auch gerade Elemente sein. In diesem Fall kann es vorteilhaft sein, wenn die Hülle des Einführelements entlang des Umfangs eine variierende Elastizität aufweist, beispielsweise in der Art eines Einführelements wie in der EP 2 676 641 A2 beschrieben ist.

Gemäß einer günstigen Ausgestaltung kann das Längselement elastisch verbiegbar sein. Das Längselement kann die gebogene Hülle durch seine Rückstellkraft und seine Stabilität in Längsrichtung stabilisieren.

Gemäß einer günstigen Ausgestaltung kann wenigstens eines der Elemente als Ringelement ausgebildet sein, das in radialer Richtung elastisch aufweitbar ist. Vorteilhaft ergibt sich ein Einführelement, welches reversibel aufweitbar ist. Dadurch, dass das Einführelement nach Passage beispielsweise des in den menschlichen und/oder tierischen Körper durch das Einführelement hindurch geschleusten Implantats wieder seinen Durchmesser verringert, wird die Gefäßbelastung des Körpers reduziert.

Gemäß einer günstigen Ausgestaltung kann wenigstens eins der Ringelemente entlang eines geschlossenen oder fast geschlossenen Umfangs gebogenen sein, wobei die beiden Enden voneinander getrennt sind. Derartige Ringelemente sind leicht zu fertigen, und deren Abmessungen sind einfach einzustellen.

Gemäß einer günstigen Ausgestaltung kann wenigstens ein Ringelement zwei an seinem Umfang überlappende Enden aufweisen. Eine Öffnung im Umfang ist somit überdeckt.

Gemäß einer günstigen Ausgestaltung kann wenigstens ein Ringelement am Umfang einen Spalt aufweisen, so dass die Enden am Umfang beabstandet sind. Insbesondere können gemäß einer günstigen Ausgestaltung wenigstens zwei Ringelemente konzentrisch angeordnet sein. Dies ist besonders zweckmäßig bei Ringelementen, die im Umfang einen Spalt aufweisen. Denkbar ist jedoch auch eine konzentrische Anordnung von Ringelementen mit überlappenden Enden.

Gemäß einer günstigen Ausgestaltung können die Elemente und/oder das Längselement metallisch sein. Das Einführelement weist eine gute Biegbarkeit sowie eine sehr gute "Trackability" im Körper auf, d.h. das Einführelement lässt sich durch die metallischen Bestandteile leicht im Körper auffinden, auch wenn es von außen nicht sichtbar ist.

Gemäß einer günstigen Ausgestaltung kann die Hülle ein Polyamid-Elastomer und/oder Polyurethan und/oder Silikon aufweisen. Dies sind geeignete Materialien für flexible Schleusen.

Gemäß einer günstigen Ausgestaltung kann die Hülle in radialer Richtung aus einem Innenteil und einem Außenteil zusammengesetzt sein, zwischen denen das Gerüst angeordnet ist. Dies erlaubt eine günstige Ausgestaltung der Hülle, bei der wünschenswerte Eigenschaften im Innenbereich für die Passage des Implantats und des Katheters durch das Einführelement hindurch und im Außenbereich für das Einführen des Einführelements in den Körper nahezu unabhängig voneinander gestaltet werden können.

Gemäß einer günstigen Ausgestaltung kann das Innenteil ein reibungsarmes Polymer umfassen, insbesondere PTFE, insbesondere Teflon.

Gemäß einer günstigen Ausgestaltung kann das Außenteil ein thermoplastisches Polymer umfassen, insbesondere ein Polyetherblockamid.

Gemäß einer günstigen Ausgestaltung kann eine reibungsvermindernde Beschichtung vorgesehen sein. Die Beschichtung kann am Innendurchmesser und/oder am Außendurchmesser des Einführelements vorgesehen sein. Dies erleichtert die Handhabung des Einführelements unter Einsatzbedingungen.

Nach einem weiteren Aspekt der Erfindung wird eine Einführvorrichtung zum Einführen eines medizinischen Implantats in einen menschlichen und/oder tierischen Körper vorgeschlagen, umfassend zumindest ein äußeres Einführelement, wobei das äußere Einführelement eine Hülle mit einer Längsachse aufweist, wobei ein Gerüst mit wenigstens bereichsweise quer zur Längsachse angeordneten und entlang der Längsachse aufeinander folgenden Elementen in die Hülle integriert ist, welches Gerüst eine höhere Härte aufweist als die Hülle. Unter Einsatzbedingungen wird die Handhabung der Einführvorrichtung erleichtert.

Die Erfindung ist nachfolgend beispielhaft, anhand von in Zeichnungen dargestellten Ausführungsbeispielen, näher erläutert. Es zeigen in schematischer Darstellung:
- Fig. 1: eine Ansicht einer Einführvorrichtung nach einem Ausführungsbeispiel der Erfindung mit einem Einführelement mit einem Gerüst, durch welches ein Implantat in einen menschlichen und/oder tierischen Körper eingeschleust wird;
- Fig. 2: eine Draufsicht auf ein Gerüst mit geraden Elementen, die mit einem Längselement verbunden sind; und
- Fog. 3: ein gebogenes Gerüst mit Ringelementen und einem die Ringelemente verbindenden Längselement.
- Fig. 4: in Seitenansicht ein Gerüst mit Ringelementen, die mittels eines Längselements verbunden sind;
- Fig. 5: in Seitenansicht ein Einführelement mit Hülle und angedeutetem Gerüst in der Hülle;
- Fig. 6: ein Querschnitt durch das Einführelement aus Figur 5;
- Fig. 7: in Draufsicht ein Detail eines Gerüsts nach einer Ausgestaltung der Erfindung, bei der aufweitbare Ringelemente mit Spalt im Umfang konzentrisch so angeordnet sind, dass die Spalte im Umfang abgedeckt sind;
- Fig. 8: in Draufsicht ein Detail eines Gerüsts nach einer Ausgestaltung der Erfindung, mit einem aufweitbaren Ringelement mit überlappenden Enden, so dass ein Spalt im Umfang überdeckt ist;

In den Figuren sind funktionell gleiche oder gleich wirkende Elemente oder Komponenten jeweils mit denselben Bezugszeichen beziffert. Die Figuren sind schematische Darstellungen der Erfindung. Sie bilden nicht spezifische Parameter der Erfindung ab. Weiterhin geben die Figuren lediglich typischen Ausgestaltungen der Erfindung wieder und sollen die Erfindung nicht auf die dargestellten Ausgestaltungen beschränken.

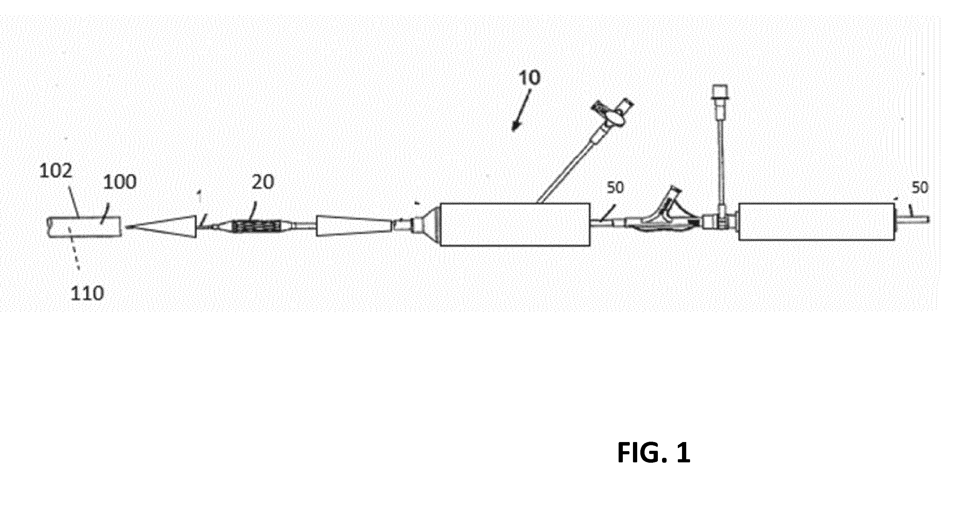

Figur 1 zeigt eine Ansicht einer Einführvorrichtung 10 nach einem Ausführungsbeispiel der Erfindung mit einem Einführelement 100, durch welches ein an einem Katheter 50 der Einführvorrichtung befestigtes Implantat 20 in einen menschlichen und/oder tierischen Körper eingeschleust wird. Das Einführelement 100 weist eine Hülle 102 auf, in der ein Gerüst 110 integriert ist, das eine höhere Härte aufweist als die Hülle 102. Hierdurch erhält das Einführelement eine bessere Stabilität in Richtung seiner Längsachse und ist stabiler gegen Kompression und Zug, was beim Einschleusen und Ausschleusen der Einführvorrichtung 10 vorteilhaft ist.

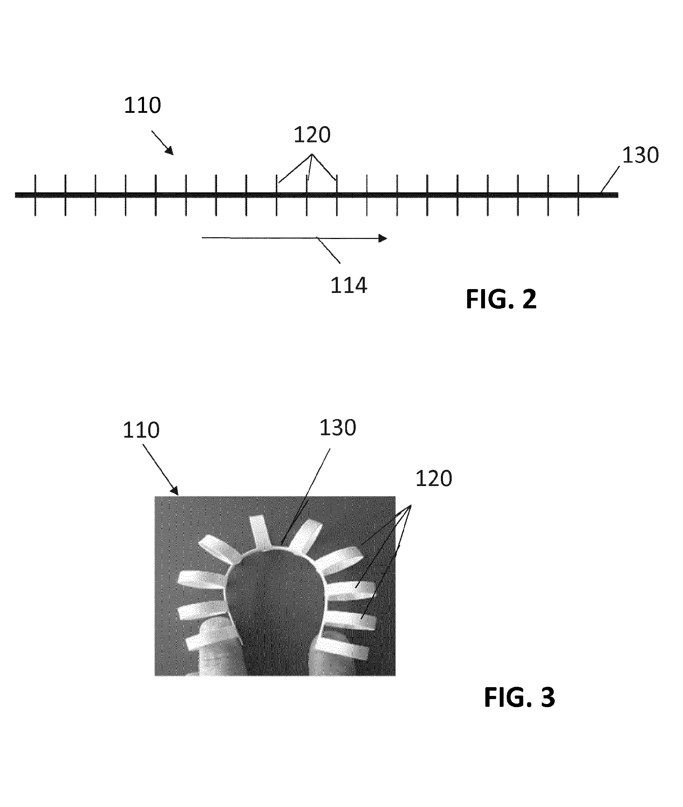

Ein Beispiel eines Gerüsts 110 ist in Figur 2 als Draufsicht dargestellt. Wie in Figur 2 zu erkennen ist, sind die Elemente 120 quer zur Längsachse 114 und aufeinander folgend entlang der Längsachse 114 angeordnet und mit einem Längselement 130 verbunden, das sich in Richtung der Längsachse 114 erstreckt. Dabei ist die Längsachse 114 die Längserstreckung des Einführelements 100 im Ruhezustand. Im Einsatz kann das Einführelement 100 gebogen sein, um z.B. einen Zugang für die Einführvorrichtung 10 in ein Körpergefäß zu ermöglichen. Die Elemente 120 können gerade ausgebildet sein oder als Ringelemente oder andere, gebogene Querschnitte aufweisen.

Figur 3 zeigt die Flexibilität des Gerüsts 110, wenn das Längselement 130 gebogen ist. Das Längselement 130 stabilisiert das Einführelement 100 auch im gebogenen Zustand gegen Zug und Kompression.

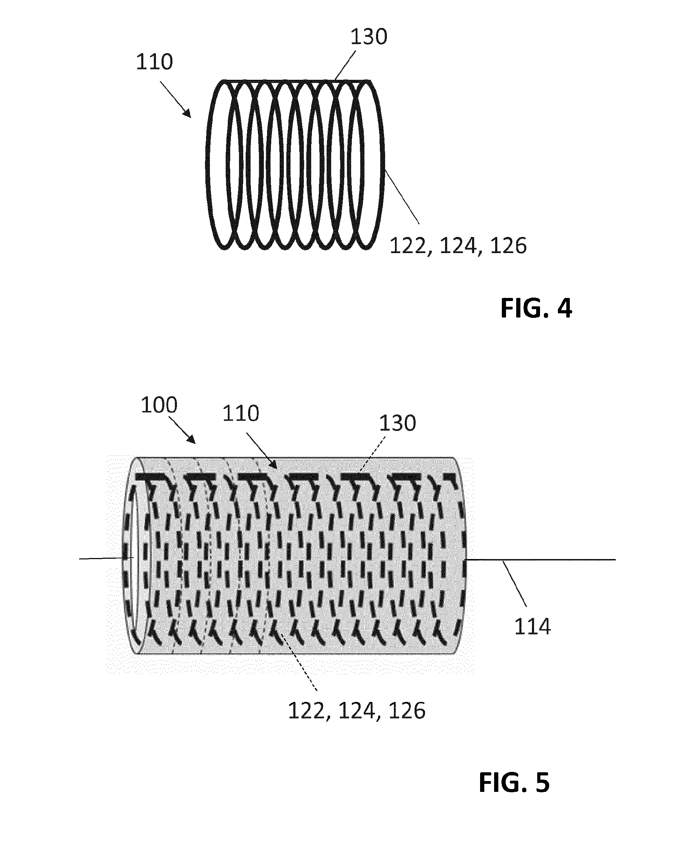

Figur 4 zeigt in Seitenansicht ein Gerüst 110 mit als Ringelemente 122, 124, 126 ausgebildeten Querelementen 120, die mittels eines Längselements 130 verbunden sind. Die Ringelemente 122, 124, 126 sind bevorzugt elastisch aufweitbar.

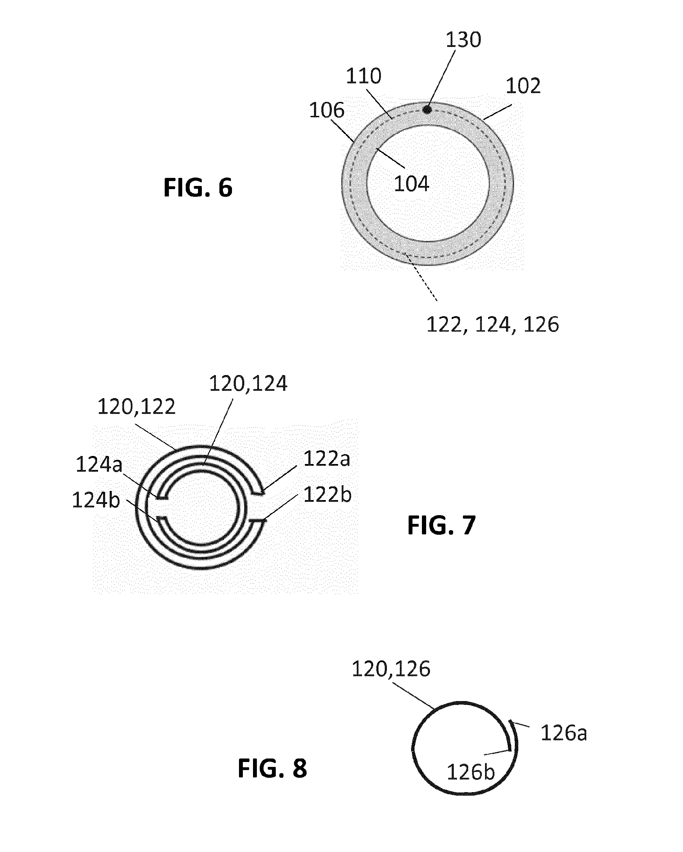

Figur 5 zeigt in Seitenansicht ein Einführelement 100 mit einer Hülle 102 und angedeutetem Gerüst 110 in der Hülle 102. Figur 6 zeigt eine Draufsicht auf einen Querschnitt durch das Einführelement 100 aus Figur. Das Längselement 130 erstreckt sich in Richtung der Längsachse 114. Bei einer Längsverbiegung des Einführelements 100 entspricht die Längsachse 114 der Sehne der Hülle 102.

Die Hülle 102 ist in radialer Richtung aus einem Innenteil 104 und einem Außenteil 106 zusammengesetzt, zwischen denen das Gerüst 110 angeordnet ist. Das Innenteil 104 umfasst ein reibungsarmes Polymer, insbesondere PTFE, wie z.B. Teflon, während das Außenteil 106 ein thermoplastisches Polymer umfasst, insbesondere ein Polyetherblockamid, wie z.B. Pebax der Firma Arkema. Alternativ oder zusätzlich kann innen und/oder außen auch eine reibungsvermindernde Beschichtung mit einem hydrophilen oder hydrophoben Material vorgesehen sein (nicht dargestellt).

Die Figuren 7 und 8 zeigen beispielhaft Elemente 120, die als elastisch aufweitbare, offene Ringelemente 122, 124, 126 ausgebildet sind. Damit kann vorteilhaft ein als Schleuse ausgebildetes Einführelement 100 mit einem elastischen radial-expandierbaren Design dargestellt werden.

Figur 7 zeigt dabei in Draufsicht ein Detail eines Gerüsts 110 nach einer Ausgestaltung der Erfindung, bei der aufweitbare Ringelemente 122, 124 mit Spalt im Umfang konzentrisch angeordnet sind. Die Ringelemente 122, 124 sind entlang eines fast geschlossenen Umfangs so gebogen, dass jeweils im Umfang ein Spalt ausgebildet ist und die Enden 122a, 122b bzw. 124a, 124b zueinander beabstandet sind. Die Spalte im Umfang eines Ringelements 122, 124 sind vom anderen Ringelement 124, 122 abgedeckt.

Eine andere Variante ist in Figur 8 dargestellt, bei der ein Ringelement 126 zwei an seinem Umfang überlappende Enden 126a, 126b aufweist.

In einem Gerüst 110 können auch verschiedenartige Ringelemente 122, 124, 126 miteinander kombiniert sein. Denkbar ist auch ein Gerüst 110, bei dem gerade und gebogene Elemente 120 miteinander kombiniert sind. Das Gerüst 110 ist vorzugsweise aus Metall gebildet, z.B. aus einem Federstahl.

Die Dimensionen der Hülle 102 und des Gerüsts 110 können bei der Herstellung leicht variiert werden. Beispielhaft sind Werte für eine übliche Schleuse angegeben: ein Ringelement 122, 124, 126 aus Stahl kann mit einem Durchmesser mit 4 mm vorgesehen sein, der auf z.B.6 mm aufgeweitet werden kann. Die radiale Dicke des Ringelements 122, 124, 126 kann bei 0,02 mm liegen, während die axiale Erstreckung bei z.B. 3 mm liegen kann. Der Abstand zwischen axial benachbarten Ringelementen 122, 124, 126 kann 3 mm betragen. Das Längselement kann aus Federstahl gebildet sein mit einem Durchmesser von z.B. 0,01 mm. Das Gerüst 110 kann beschichtet sein, beispielsweise mit einer 0,03 mm dicken Pebax-Schicht. Vorteilhaft besteht die Hülle 102 ein Polyamid-Elastomer und/oder Polyurethan und/oder Silikon.

Diese Erfindung erlaubt ein Design für ein schlauchartiges Einführelement mit flexibler Biegbarkeit und zugleich eine hohe axiale Festigkeit des Einführelements.

## Patentansprüche

1. Einführelement (100), welches zum Zusammenwirken mit einer Einführvorrichtung (10) zum Einführen eines medizinischen Implantats (20) in einen menschlichen oder tierischen Körper ausgebildet ist, und welches eine Hülle (102) mit einer Längsachse (114) aufweist, **dadurch gekennzeichnet, dass** ein Gerüst (110) mit wenigstens bereichsweise quer zur Längsachse (114) angeordneten und entlang der Längsachse (114) aufeinander folgenden Elementen (120) in die Hülle (102) integriert ist, wobei die Elemente (120) entlang der Längsachse (114) mit einem Längselement (130) verbunden sind, und welches Gerüst (110) eine höhere Härte aufweist als die Hülle (102).

2. Einführelement nach Anspruch 1, **dadurch gekennzeichnet, dass** das Längselement (130) elastisch verbiegbar ist.

3. Einführelement nach Anspruch 1 oder 2, **dadurch gekennzeichnet, dass** wenigstens eines der Elemente (120) als Ringelement (122, 124, 126) ausgebildet ist, das in radialer Richtung elastisch aufweitbar ist.

4. Einführelement nach Anspruch 3, **dadurch gekennzeichnet, dass** wenigstens eines der Ringelemente (122, 124, 126) entlang eines geschlossenen oder fast geschlossenen Umfangs gebogenen ist, wobei die beiden Enden (122a, 122b; 124a, 124b; 126a, 126b) voneinander getrennt sind.

5. Einführelement nach Anspruch 3 oder 4, **dadurch gekennzeichnet, dass** wenigstens ein Ringelement (126) zwei an seinem Umfang überlappende Enden (126a, 126b) aufweist.

6. Einführelement nach einem der Ansprüche 3 bis 5, **dadurch gekennzeichnet, dass** wenigstens ein Ringelement (126) am Umfang einen Spalt aufweist, so dass die Enden (122a, 122b; 124a, 124b) am Umfang beabstandet sind.

7. Einführelement nach einem der Ansprüche 3 bis 6, **dadurch gekennzeichnet, dass** wenigstens zwei Ringelemente (122, 124) konzentrisch angeordnet sind.

8. Einführelement nach einem der vorhergehenden Ansprüche, **dadurch gekennzeichnet, dass** die Elemente (120) und/oder das Längselement (130) metallisch sind.

9. Einführelement nach einem der vorhergehenden Ansprüche, **dadurch gekennzeichnet, dass** die Hülle (102) ein Polyamid-Elastomer und/oder Polyurethan und/oder Silikon aufweist

10. Einführelement nach einem der vorhergehenden Ansprüche, **dadurch gekennzeichnet, dass** die Hülle (102) in radialer Richtung aus einem Innenteil (104) und einem Außenteil (106) zusammengesetzt ist, zwischen denen das Gerüst (110) angeordnet ist.

11. Einführelement nach Anspruch 10, **dadurch gekennzeichnet, dass** das Innenteil (104) ein reibungsarmes Polymer umfasst, insbesondere PTFE.

12. Einführelement nach Anspruch 10 oder 11, **dadurch gekennzeichnet, dass** das Außenteil (102) ein thermoplastisches Polymer umfasst, insbesondere ein Polyetherblockamid.

13. Einführelement nach einem der vorhergehenden Ansprüche, **gekennzeichnet durch** eine reibungsvermindernde Beschichtung.

14. Einführvorrichtung (10) zum Einführen eines medizinischen Implantats (20) in einen menschlichen und/oder tierischen Körper, umfassend zumindest ein äußeres Einführelement (100), wobei das äußere Einführelement (100) nach einem der vorhergehenden Ansprüche.
